# EUROPEAN PATENT SPECIFICATION

(11) **EP 2 404 576 B1**
(45) Date of publication and mention of the grant of the patent: **30.10.2019**
(21) Application number: 10748665.6
(22) Date of filing: 25.02.2010
(51) Int. Cl.: A61F 2/82

(54) **MARKER AND STENT**
MARKER UND STENT
REPÈRE ET ENDOPROTHÈSE

(30) Priority: 03.03.2009 JP 2009048836
(43) Date of publication of application: 11.01.2012
(73) Proprietor: MANI, INC., Utsunomiya Tochigi 321-3231 (JP)
(72) Inventor: YOKOI, Yoshihiko, Tokyo 168-0072 (JP); HASHIMOTO, Yasushi, Utsunomiya-shi Tochigi 321-3231 (JP); SAITO, Akira, Utsunomiya-shi Tochigi 321-3231 (JP)
(74) Representative: Westphal, Mussgnug & Partner Patentanwälte mbB
(86) International application number: PCT/JP2010/052965
(87) International publication number: WO 2010/101070

(56) References cited:
- EP-A1- 2 044 907
- WO-A1-2008/015873
- WO-A1-2008/015873
- JP-A- 2001 231 868
- JP-A- 2004 097 382
- JP-A- 2004 097 382
- US-A1- 2004 167 619
- US-A1- 2005 149 171
- US-B1- 6 890 350

## Description

### TECHNICAL FIELD

The present invention relates to a marker which is advantageous for confirming the position of a stent within a human body by a recognition means including, e.g., an X-ray imaging apparatus in a process of mounting the stent within the human body, and to the stent provided with the marker.

### BACKGROUND ART

For instance, when an aneurysm is treated, a stent graft in which a graft is applied onto a cylindrical instrument called a stent has been often used. The stent graft is accommodated in a sheath with a diameter of the stent graft being decreased, is conveyed to an affected part to be treated, is removed from the sheath in the affected part, and is mounted with the diameter being increased. For this reason, blood flows through the inside of the stent graft mounted inside the aneurysm, so that the pressure of the blood can be prevented from acting on the aneurysm.

When the stent graft is mounted in an affected part, it is necessary to accurately set its position. Therefore, during an operation, the sheath is allowed to reach a target affected part, and thereafter, the stent graft is removed from the sheath while its position is recognized. In this way, when the stent graft is mounted in the target affected part, it is essential to recognize the position of the stent graft within a human body, so that the stent graft is provided with a marker. The marker is typically provided on the stent, not on the graft.

The marker is typically configured of, e.g., an X-ray non-transmission section in which gold plating or gold foil is applied onto part of a stent main wire or part of a strut, which configures the stent. Then, X-ray imaging is performed to recognize the X-ray non-transmission section (marker) , so that the position of the stent within a human body can be confirmed.

However, the X-ray non-transmission section (marker) onto which gold plating or gold foil is applied can be separated under the influence of a blood flow over a long period. Therefore, there has been proposed a stent in which a portion onto which gold plating or gold foil is applied is protected by a pipe to prevent separation and entering into a blood flow even in the case of separation (for instance, see Patent Document 1).

### RELATED ART DOCUMENT

### PATENT DOCUMENT

Patent Document 1: Japanese Patent Application Laid-Open (JP-A) No. 2004-097382

### DISCLOSURE OF THE INVENTION

### Problems to be Solved by the Invention

As described in Patent Document 1, the portion onto which gold plating or gold foil is applied, which configures the X-ray non-transmission section (marker), is protected by the pipe, so that the separated gold plating or gold foil can be prevented from mixing into a blood flow. However, the operation of applying gold plating or gold foil onto part of the stent main wire or part of the strut is not easy.

That is, although the diameter of the stent main wire and the diameter of the strut are different from each other, they are typically about 0.6 mm, and the operation of applying gold plating or gold foil onto part of such thin wire is a very fine operation, is troublesome, and has poor operability.

In addition, gold plating or gold foil is applied onto the entire stent or the entire strut in order to improve the operability, resulting in increase of the cost and a possibility of separation, which is unpreferable.

Stents with a marker for confirming a position of the stent formed of wires within a human body, wherein the stent has a stent main wire formed in a zigzag pattern and a pipe, which is made of a raw material which can be recognized by a recognition means and is fitted to a zigzag-shaped turn-back section of the stent main wire and/or vicinity of the turn-back section are known for example from US US 2005/149171 A1, US 6 890 350 B1, US 2004/167619 A1, JP 2004 097382, WO 2008/015973 A1 and EP 2 044 907 A1.

An object of the present invention is to provide a marker which is formed without applying gold plating or gold foil thereonto and can be recognized by a recognition means, and a stent provided with the marker.

### Means for Solving the Problems

To address the above problems, a marker for confirming a position of a stent formed of wires within a human body, wherein the stent has a stent main wire formed in a zigzag pattern, and a pipe, which is made of a raw material which can be recognized by a recognition means is fitted to a zigzag-shaped turn-back section of the stent main wire and/or a vicinity of the turn-back section.

In the marker, preferably, the turn-back section has a curved section formed in a curved shape, and a parallel section which is continuous with the curved section, and the pipe is fitted to the parallel section.

In addition, in the marker, preferably, the pipe is fitted to the stent main wire configuring the zigzag-shaped turn-back section and/or the turn-back section vicinity, and/or is fitted so as to hold the stent main wires together.

In addition, in the marker, more preferably, the pipe is fitted so as to hold the stent main wires configuring the zigzag-shaped turn-back section and/or the turn-back section vicinity together by including a different wire arranged so as to extend from the turn-back section and/or the turn-back section vicinity.

In addition, in any one of the markers, preferably, the pipe is fitted so as to be fixed to at least one wire configuring the stent.

In addition, a stent according to the present invention, which has a stent main wire formed in a zigzag pattern, wherein any one of the markers is provided in the zigzag-shaped turn-back section and/or the turn-back section vicinity.

### Effects of the Invention

In the marker according to the present invention, the stent has a stent main wire formed in a zigzag pattern, and a pipe, which is made of a raw material which can be recognized by a recognition means is fitted to a zigzag-shaped turn-back section and/or a turn-back section vicinity of the stent main wire, so that a satisfactory marker function corresponding to a recognition method by the recognition means can be exhibited.

The marker of the present invention can be formed by the easy operation of fitting the pipe to the zigzag-shaped turn-back section and/or the turn-back section vicinity of the stent main wire configuring the stent, and can be formed without requiring the troublesome operation of applying gold plating or gold foil onto the stent.

In addition, the pipe is fitted to the zigzag-shaped turn-back section and/or the turn-back section vicinity of the stent main wire, so that the fitted pipe reduces a gap between two stent main wires which are located close to each other, whereby the marker function can be exhibited more satisfactorily.

In addition, the turn-back section has a curved section formed in a curved shape, and a parallel section which is continuous with the curved section, and the pipe is fitted to the parallel section, so that the pipe caulking operation can be performed stably. Further, the pipe is hard to move from the parallel section to a straight line section of the stent main wire.

In addition, the pipe is fitted to the stent main wire configuring the zigzag-shaped turn-back section and/or the turn-back section vicinity, and/or is fitted so as to hold the stent main wires together, so that the pipe cannot be removed from the stent main wire. In addition, a wall having a thickness twice the wall thickness of the pipe is formed between two stent main wires configuring the turn-back section. Therefore, this wall can function as the marker for mounting the stent.

In addition, the pipe is fitted so as to hold the stent main wires configuring the zigzag-shaped turn-back section and/or the turn-back section vicinity together by including a different wire arranged so as to extend from the turn-back section and/or the turn-back section vicinity, so that the attaching strength with respect to the stent main wires of the different wire including a strut or a stabilizer hook configuring the stent can be improved, and the marker including the different wire can function more satisfactorily.

In addition, the pipe is fitted so as to be fixed to at least one wire configuring the stent, so that the pipe cannot be removed from the wire when the stent is mounted in an affected part, and can reliably function as the marker.

In addition, in a stent according to the present invention, the marker according to any one of claims of the present invention is provided in the zigzag-shaped turn-back section and/or the turn-back section vicinity, so that the position of the stent within a human body can be reliably recognized by the recognition means when the operation of mounting the stent within the human body is performed.

### BRIEF DESCRIPTION OF THE DRAWINGS

Fig. 1 is a diagram for explaining the configuration of a marker according to a first example.
Fig. 2 is a diagram for explaining the configuration of a stent.
Fig. 3 is a diagram for explaining the configuration of a marker according to a second example.
Fig. 4 is a diagram for explaining the configuration of a marker according to a third example.

### Description of Reference Numerals

- A, B: Marker
- C: Stent
- D: Loop stent
- E: Turn-back section
- 1: Stent main wire
- 1a: Curved section
- 1b: Parallel section
- 1c: Straight line section
- 2: Strut
- 3: Connection pipe
- 4: Stabilizer hook
- 10 to 12: Pipe

### Best Modes for Carrying Out the Invention

Hereinafter, most preferred embodiments of a marker according to the present invention and a stent using the marker will be described.

The marker according to the present invention is used for confirming the position within a human body of a stent formed of wires by a recognition means. In particular, the stent has a stent main wire formed in a zigzag pattern, and a pipe, which is made of a raw material which can be recognized by the recognition means is fitted to a zigzag-shaped turn-back section and/or a turn-back section vicinity of the stent main wire, so that the marker is configured of the pipe or the wire configuring the pipe and the stent.

The wire configuring the stent is required to be of a material which cannot affect a human body. Examples of such material include metals such as stainless steel, titanium, and titanium alloys, which can be used selectively. In particular, examples of the materials which cannot affect a human body even if the stent is mounted within the human body for a long period include SUS316L and SUS304, which are austenitic stainless steel. In particular, SUS316L is typically used as implant stainless steel, and can be used preferably as the stent.

The pipe is fitted to a portion at which at least two wires configuring the stent are located close to each other, and is used for confirming the position of the stent within a human body by the recognition means. For this reason, the pipe is formed of a raw material which can be recognized by the recognition means. That is, the pipe is made from a raw material corresponding to a recognition method by the recognition means .

In the present invention, it suffices that the recognition means has the function of recognizing the marker when the stent provided with the marker is mounted within a human body, and does not particularly limit its configuration. Examples of such recognition means include an X-ray imaging apparatus and magnetic resonance imaging (MRI). Any of these can be used as the recognition means for mounting the stent, or as the recognition means for confirming the position of the mounted stent.

When the X-ray imaging apparatus is used as the recognition means, the pipe is required to be of a material which becomes a sufficient obstacle to emitted X-ray transmission. Examples of such material include metals such as gold, platinum, and stainless steel. However, when noble metal such as gold and platinum is used, the issue of increasing the cost can arise. In addition, when the material of the wire configuring the stent and the material of the pipe are different, electrocorrosion might occur. Therefore, preferably, the material of the pipe is the same as that of the wire configuring the stent or cannot cause electrocorrosion.

In addition, when MRI is used as the recognition means, synthetic resin and non-magnetic metal can be used as the pipe. Examples of such metals include gold, platinum, and nickel titanium alloys. However, when noble metal such as gold and platinum is used, the issue of increasing the cost can arise.

The length and wall thickness of the pipe are not particularly limited, and are preferably set according to the diameter of the wire configuring the stent. The pipe is not always required to function singly as the marker, and it suffices that the pipe functions as the marker together with the wire configuring the stent. Therefore, the size of the pipe is preferably set as needed according to the wire to which the pipe is to be fitted, in diameter and number.

In addition, the shape of the pipe is not limited, and it suffices that the pipe has shapes which can be fitted to the wire configuring the stent. Examples of such shapes include cylindrical, elliptical, triangular, and square shapes. The pipes having these shapes can be adopted selectively.

The pipe is fitted to the zigzag-shaped turn-back section and/or the turn-back section vicinity of the stent main wire. In that case, in which state the pipe is fitted is not limited, and it suffices that the pipe is fitted in a pipe shape to one or more wires at a position at which at least two wires are located close to each other.

However, when the pipe is fitted to at least one of two wires configuring the stent, the stent can move from the mounted portion when mounted in an affected part. For this reason, the pipe is preferably fixed and fitted to the wire configuring the stent by welding and plastic working.

Since the stent of the present invention is a loop stent configured by forming the stent main wire in a zigzag pattern and joining ends thereof, two stent main wires are located close to each other in the zigzag-shaped turn-back section.

In this case, the cylindrical pipe having an inside diameter larger than the outside diameter of the stent main wire is fitted, and the pipe is arranged and fitted to a portion at which at least two stent main wires are located close to each other in the zigzag-shaped turn-back section.

In addition, the pipe is fitted to each of two stent main wires which are located close to each other in the turn-back section and/or the turn-back section vicinity, so that the marker can be formed of the fitted pipe and the wire. In the marker configured in this way, while the stent is conveyed to a target affected part or when the stent is removed from a sheath in the target affected part, the pipe can exhibit a reliable marker function without being removed from the stent even when the pipe moves along the stent main wire.

In addition, the pipe can also serve both as a connection pipe for connecting a strut or a stabilizer hook to the stent main wire and a marker pipe to be the marker. In this case, the pipe is required to have dimensions in consideration of accommodation of the strut or the stabilizer hook in addition to the stent main wire. However, the connection pipe is passed through the stent main wire to prevent falling, and has a size limited to some extent. Therefore, desirably, the connection pipe and the marker pipe are formed separately.

Further, the pipe may be fitted from the top side of the turn-back section at which at least two stent main wires are located close to each other in the stent configured as described above (or from the outside of the stent) across the two wires so as to hold the wires together. In this case, the marker can be formed of the pipe fitted so as to hold the two stent main wires together, or of the pipe and the two stent main wires. In the marker configured in this way, while the stent is conveyed to a target affected part or when the stent is removed from the sheath in the target affected part, the pipe can be removed from the stent, so that the pipe is required to be fixed strongly.

However, the marker formed at the turn-back section and/or the turn-back section vicinity, in which in addition to two stent main wires which are located close to each other in the zigzag-shaped turn-back section and/or the turn-back section vicinity, a different wire such as the strut or the stabilizer hook is extended and arranged, cannot be fallen, which is advantageous. That is, the pipe is fitted so as to hold three wires together, that is, two stent main wires in the turn-back section and/or the turn-back section vicinity and the wire such as the strut or the stabilizer hook, so that even when removed, the fitted pipe is caught by the different wire or the loop stent. The marker cannot be removed from the stent.

### First Example

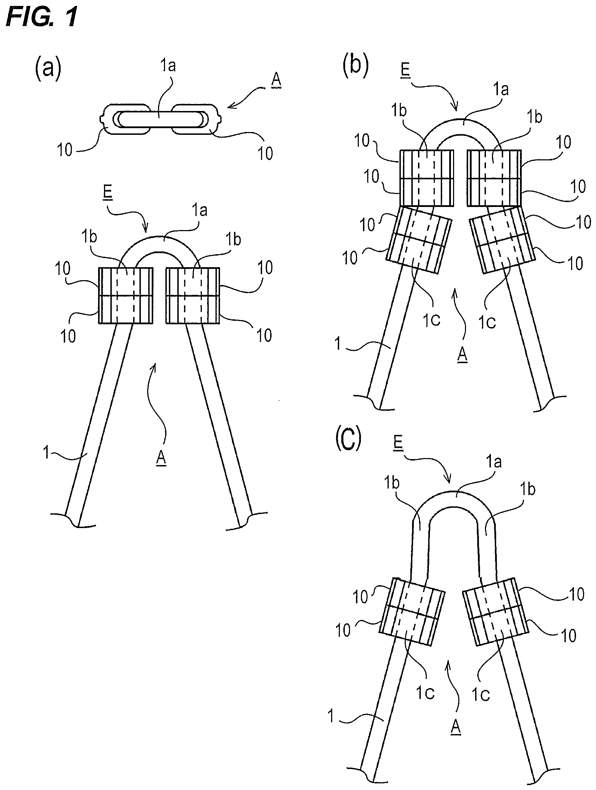

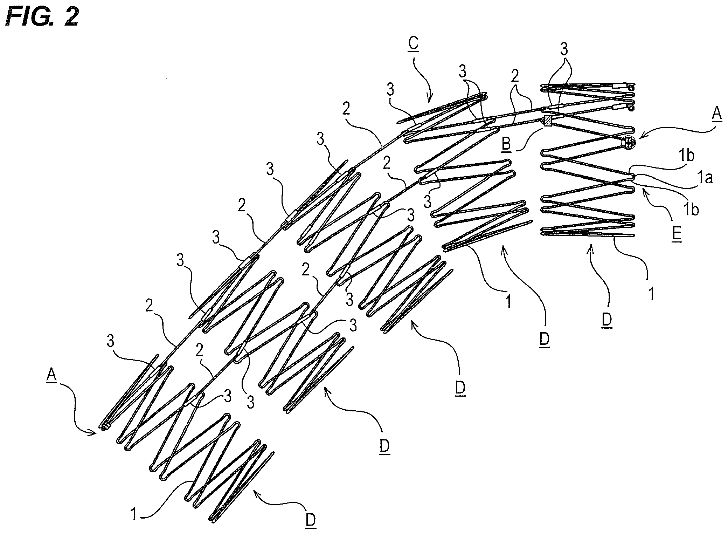

Next, the configurations of a marker according to a first example and a stent provided with the marker will be described with reference to the drawings. Fig. 1 is a diagram for explaining the configuration of the marker according to the first example. Fig. 2 is a diagram for explaining the configuration of the stent.

First, the overall configuration of a stent C will be described with reference to Fig. 2. In the stent C, plural (five in this example) loop stents D in which stent main wires 1 are formed in a zigzag pattern and ends thereof are welded are connected via struts 2.

The stent main wire 1 configuring each of the loop stents D uses austenitic stainless steel, in particular, a wire of SUS316L typically provided as implant stainless steel. Then, this wire is cold-drawn to extend its tissue in a fiber shape for work hardening to improve the mechanical properties. The loop stent D is made from such material, so that excellent suitability to a human body and an appropriate extension force can be exhibited.

When the stent main wire 1 is formed in a zigzag pattern to configure the loop stent D, a turn-back section E of the stent main wire 1 is preferably formed so as to have a curvedly formed curved section 1a of its front end portion, and a parallel section 1b which is continuous with the curved section 1a.

The turn-back section E of the loop stent D is configured in this way, so that spring properties can be exhibited by the turn-back section E, and that when the stent C is accommodated in a sheath with a diameter of the stent C being decreased or is removed from the sheath with the diameter being increased, smooth diameter decrease and increase can be realized without concentrating a force onto the turn-back section. Further, when the marker is provided in the turn-back section E in at least the front end portion of the stent C, the pipe can be fitted using the parallel section 1b, so that the caulking operation can be performed stably. In addition, the pipe is hard to move from the parallel section to a straight line section of the stent main wire.

The individual loop stents D are formed in a substantially cylindrical shape corresponding to the thickness of an affected part in which they are to be mounted. Then, these loop stents D are arrayed according to the shape of the affected part in which they are to be mounted, and are connected via the struts 2 so as to hold the respective arrayed positions, thereby configuring the stent C.

The stent main wire 1 and the strut 2 configuring the loop stent D are connected using a connection pipe 3. The connection configuration of the stent main wire 1 and the strut 2 using the connection pipe 3 is the same as that described in Patent Document 1. That is, the connection pipe 3 is configured of plural relatively short pipes, and is fitted and caulked in the state that the end of the strut 2 is overlapped with the stent main wire 1.

At least the front end portion of the stent C configured by connecting the loop stents D via the strut 2, as described above, is provided with a marker A according to the first example. However, the marker A is not always provided only on the front end portion of the stent C, and may be provided in the rear end portion or a predetermined set position of the stent C. In this example, the marker A is provided in the front or rear end portion of the stent C.

As shown in Fig. 1 (a) , the marker A according to the first example is configured by fitting and caulking the pipe 10 to each of two stent main wires 1 which are located close to each other and configure the turn-back section E of the loop stent D.

When the loop stent D is configured, the pipe 10 is fitted to the stent main wire 1 when the stent main wire 1 is formed in a zigzag pattern. For this reason, the pipe 10 has a length which can smoothly pass it through the turn-back section E. Then, plural pipes 10 are fitted and caulked to each of the stent main wires 1 so as to have a length which can exhibit a sufficient function as the marker A.

The pipe 10 configured of a wire made of austenitic stainless steel which is a material similar to the stent main wire 1. In particular, since in this example, the stent main wire 1 uses SUS316L, preferably, the pipe 10 also uses the same material SUS316L, or SUS304 . In addition, the pipe 10 is not required to be cold-drawn for work hardening.

In this example, the pipe 10 is set to a length of about 0.8 mm, and two pipes 10 having this length are arranged longitudinally. In addition, the diameter of the stent main wire 1 is set to about 0.45 mm, and according to this, the pipe 10 is set to an inside diameter of about 0.7 mm, an outer diameter of about 1.3 mm, and a wall thickness of about 0.3 mm.

Then, when the pipe 10 is caulked, the thickness of the pipe 10 and the stent main wire 1 becomes about 0.95 mm and the width of the pipe 10 becomes about 1.6 mm, so that a gap between the ends of the caulked pipes 10 is set so as to be eliminated to the extent possible.

Therefore, the marker A, which is made of stainless steel and has a length of about 1.6 mm, a width of about 3.6 mm, and the largest thickness of about 0.95 mm, is configured in the turn-back section E of the loop stent D. When inserted into a human body, the marker A has a thickness in which it can sufficiently function as a non-transmission section to an X-ray emitted onto the human body.

In particular, to allow the marker A to function as the X-ray non-transmission section using stainless steel, its thickness becomes an issue, and like this example, the marker A having a thickness of about 0.95 mm can sufficiently exhibit the X-ray non-transmission section function. Preferably, the marker A whose thickness is not limited has a thickness of 0.9 mm or more at caulking in order to exhibit the X-ray non-transmission section function. In addition, the marker A with a too large thickness is thickened and it is less likely to make a diameter thereof decreased when the graft is turned back, so that the caulked marker A preferably has a thickness of about 1.0 mm or less.

In addition, as shown in Fig. 1(b), the marker A can also be configured by arranging and caulking plural pipes 10 which are continuous with the pipe 10 arranged on the parallel section 1b in the turn-back section E. That is, the length (number) of the pipes 10 used for configuring the marker A is not particularly limited, and is preferably set as needed corresponding to the arranged position of the marker A in the stent C. Further, as shown in Fig. 1(c), the marker A can also be configured by arranging and caulking the pipe 10 only on a straight line section 1c which is continuous with the parallel section 1b near the turn-back section E without arranging the pipe 10 on the parallel section 1b in the turn-back section E.

Further, since in this example, the pipe 10 has a length which pass it through the zigzag-shaped turn-back section E of the loop stent D and smooth operability, the pipe 10 has a length three or less times the diameter of the stent main wire 1. For instance, when the diameter of the stent main wire 1 is 0.45 mm, the pipe 10 has a length of about 0.5 mm to 1.35 mm, so that a smooth operation can be realized.

In the marker A configured as described above, when an X-ray imaging apparatus is adopted as the recognition means, the X-ray non-transmission section can be configured of the pipe 10 caulked to the stent main wire 1. In particular, since the pipe 10 is fitted and caulked to each of two stent main wires 1 which are located close to each other in the turn-back section E, the pipe 10 cannot be fallen off the stent C when the stent C is conveyed to a target affected part and is removed from the sheath in the affected part.

Further, although the pipe 10 is fitted to each of two stent main wires 1, which is preferable since the width of the entire marker section is increased, this configuration is not limited, and the pipe 10 is fitted to one stent main wire 1 (only one wire), which is included in the scope of the present invention.

### Second example

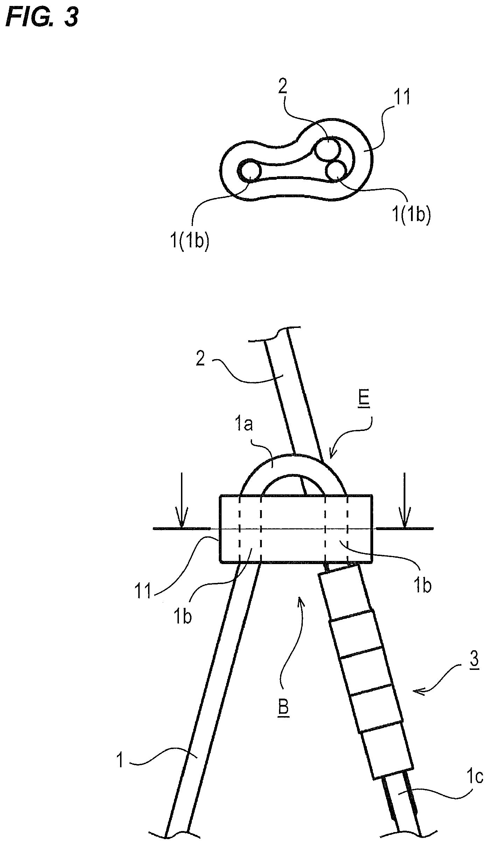

Next, the configuration of a marker according to a second example will be described with reference to the drawing. Fig. 3 is a diagram for explaining the configuration of a marker B according to the second example.

The marker B according to this example is configured by caulking a pipe 11 fitted across two stent main wires which are located close to each other in the turn-back section E of the loop stent D. In addition, the pipe 11 used in this example is made from SUS316L, as in the above example.

The pipe 11 shown in Fig. 3 is fitted across two stent main wires 1 which are located close to each other in the turn-back section E of the loop stent D from the outside of the loop stent D. Then, the pipe 11 is caulked at a position corresponding to the parallel sections 1b of the two stent main wires 1 in the turn-back section E, thereby configuring the marker B.

The marker B configured as described above is configured by caulking the pipe 11 fitted from the outside to the two stent main wires 1 which are located close to each other in the turn-back section E. For this reason, the two stent main wires 1 can be continuous by the pipe 11 without any gap, so that the marker B can be preferable for inhibiting X-ray transmission.

However, when the marker B is provided in the front end portion of the stent C, the pipe 11 can be inevitably fallen off the turn-back section E when the stent C is conveyed to a target affected part and is removed from a sheath in the affected part for mounting. For this reason, the marker B is provided in a portion in which the fitted pipe 11 cannot be fallen off the stent C even when removed from the two stent main wires 1 in the turn-back section E.

The position at which the pipe 11 cannot be fallen off the stent C is, e.g., a joining portion of the stent main wire 1 and the strut 2 of the loop stent D shown in Figs. 2 and 3. That is, as shown in Fig. 3, the end of the strut 2 is arranged along the straight line section 1c of one of the stent main wires 1. The connection pipe 3 is fitted to the straight line section 1c and the end of the strut 2, and is caulked to join the strut 2 to the stent main wire 1 via the straight line section 1c. Then, both ends of the strut 2 are joined to the stent main wires 1 of the adjacent loop stents D via the connection pipe 3, so that the two loop stents D are connected to each other.

Therefore, when the strut 2 is joined to the loop stent D, the pipe 11 is fitted to the strut 2. Next, the stent main wire 1 of the loop stent D is joined to the strut 2 via the connection pipe 3, and the pipe 11 is fitted and caulked across the two stent main wires 1 which are located close to each other in the turn-back section E, thereby configuring the marker B. Since the loop stent D is connected to each of both ends of the strut 2, the pipe 11 cannot be fallen off the stent C even when removed from the turn-back section E.

The diameter (about 0.7 mm) of the strut 2 is larger than that of the stent main wire 1. In addition, when the stent C is shrunk in diameter or is removed from the sheath so as to be grown in diameter, the strut 2 can be curved with the joining portion thereof to the connection pipe 3 as a start point. For this reason, the pipe 11 having a relatively large wall thickness is adopted. Therefore, even when the strut 2 is curved to allow a force to act on the pipe 11, the pipe 11 can resist the force.

In this example, the pipe 11 is set to an inside diameter of about 1.8 mm, an outside diameter of about 2.6 mm (a wall thickness of about 0.4 mm), and a length of about 2.0 mm.

The pipe 11 having the dimensions is fitted and caulked across the two stent main wires 1 which are located close to each other and the strut 2 in the turn-back section E of the loop stent D. At this time, as shown in the sectional view, a void of about 0.3 mm is formed without closing a portion between the two stent main wires 1 under the influence of the stent main wires 1 and the strut 2 to which the pipe 11 is fitted. In addition, one stent main wire 1 side has a thickness of about 1.7 mm according to the diameter of the stent main wire 1 and the wall thickness of the pipe 11, and the other stent main wire 1 side has a thickness of about 2.4 mm according to the diameter of the stent main wire 1, the diameter of the strut 2, and the wall thickness of the pipe 11. Although in this example, preferably, one stent main wire 1 side (one wire side) whose thickness is not limited has a thickness of 1.2 mm or more at caulking in consideration of exhibition of an X-ray non-transmission section function and application of a force which can resist the curving of the strut 2. In addition, since a too large thickness causes a difficulty of decrease in diameter, the thickness is preferably about 1.8 mm or less at caulking.

In addition, to satisfactorily exhibit the marker function, the width of the pipe 11 is also important. In this example, the pipe has a width of about 3.2 mm at caulking.

The marker B configured by caulking the pipe 11 fitted together with the strut 2 to the stent main wire 1 in the turn-back section E of the loop stent D, as described above, can exhibit a sufficient X-ray non-transmission function when an X-ray imaging apparatus is adopted as the recognition means. Third example

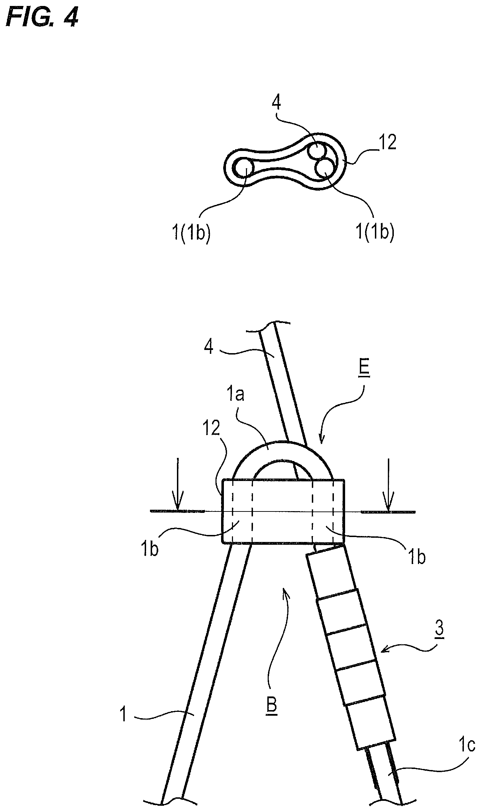

Next, the configuration of the marker B according to a third example will be described with reference to the drawing. Fig. 4 is a diagram for explaining the marker provided in the attaching portion of a stabilizer hook arranged on the front end portion of the stent. Further, this example has the same configuration as the second example except that a stabilizer hook 4 is used in place of the strut 2. For this reason, the same portions as the second embodiment are indicated by similar reference numerals, and the description will not be repeated.

The stabilizer hook 4 is arranged at the front end of the stent C, and engages the stent C with the periphery of a target affected part when the stent C is mounted in the affected part. Since a large force cannot act on the stabilizer hook 4 as compared with the strut 2, the diameter of the stabilizer hook (about 0.45 mm) is equal to or smaller than that of the stent main wire 1.

In addition, a stent graft, not shown, is applied onto the stent C. In particular, since the stent graft is turned back and applied onto the end of the stent C, the stent graft has a double thickness and a larger volume than other portions. For this reason, when a pipe 12 of this example has the same wall thickness as the pipe 11 of the second example, a disadvantage can occur when the stent graft is shrunk in diameter and is accommodated in a sheath. Therefore, it suffices that the pipe 12 has a wall thickness which satisfies the thickness to the extent that an X-ray non-transmission function can be exhibited. As numeral values, at caulking, when the thickness of one of the stent main wires 1 (one wire) is 0.5 mm or more, the X-ray non-transmission function can be exhibited, and when the thickness is about 1.2 mm or less, no disadvantage can occur at decrease in diameter.

In this example, the pipe 12 is set to an inside diameter of about 1.8 mm, an outside diameter of about 2.2 mm (a wall thickness of about 0.2 mm), and a length of about 2.0 mm.

The pipe 12 having the dimensions is fitted and caulked across the two stent main wires 1 which are located close to each other and the stabilizer hook 4 in the turn-back section E at the front end of the loop stent D arranged at the head of the stent C. At this time, as shown in the sectional view, the pipe 12 is formed with a void of about 0.2 mm without closing a portion between the two stent main wires 1 under the influence of the stent main wires 1 and the stabilizer hook 4 to which the pipe 12 is fitted. In addition, one stent main wire 1 side has a thickness of about 1.1 mm according to the diameter of the stent main wire 1 and the wall thickness of the pipe 12, and the other stent main wire 1 side has a thickness of about 1.7 mm according to the diameter of the stent main wire 1, the diameter of the stabilizer hook 4, and the wall thickness of the pipe 12.

In addition, to satisfactorily exhibit the marker function, the width of the pipe 12 is also important. In this example, the pipe 12 has a width of about 2.8 mm at caulking.

The marker B configured by caulking the pipe 12 fitted together with the stabilizer hook 4 to the stent main wires 1 in the turn-back section E of the loop stent D, as described above, can exhibit a sufficient X-ray non-transmission function when an X-ray imaging apparatus is adopted as the recognition means.

In the markers A and B configured as described above, when the X-ray imaging apparatus is adopted as the recognition means, the X-ray non-transmission section can be configured by the easy operation of fitting and caulking any one of the pipes 10, 11, and 12 to the two stent main wires 1 which are located close to each other in the turn-back section E of the loop stent D. In addition, in the markers A and B, when the stent C is conveyed to a target affected part or is mounted in the target affected part, the pipes 10, 11, and 12 cannot be fallen off the stent C even when removed from the stent main wires 1.

In the above examples, the markers A and B which adopt the X-ray imaging apparatus as the recognition means and have the X-ray non-transmission section function have been described. However, when non-magnetic austenitic stainless steel is adopted for the markers A and B, the position of them within a human body can be confirmed even when MRI is adopted as the recognition means.

As shown in Fig. 2, in the stent C according to the present invention, the marker A according to the first example is provided on at least the front end of the stent C or the marker B according to the third example is provided together with a stabilizer hook (not shown in Fig. 2) arranged at the front end of the stent C. For this reason, the markers A and B function as the X-ray non-transmission section, and X-ray imaging is performed when the stent C is mounted within a human body, so that the position of the stent C can be confirmed.

Therefore, the progress in which the sheath accommodating the stent C is operated to convey the stent C to a target affected part can be confirmed, and the position of the stent C at which the sheath reaches the target affected part and is mounted can be confirmed accurately.

In addition, when the marker B according to the second example is provided together with the strut 2 which connects the loop stent D configuring the stent C, a portion other than the front or rear end of the stent C can be confirmed.

In the examples, a connection means of the pipes 10, 11, and 12 is caulking, which is not limited. Needless to say, a welding means or the like can also be adopted.

In addition, although in the examples, the dimensions of the markers A and B are not limited, in order to allow the markers to satisfactorily function, the area of one marker (in the marker A, when two pipes 10 are provided on each stent main wire 1) is as large as possible, and when the fitted pipe has the entire width of 2.5 mm or more, a length of 1.0 mm or more, and a thickness of 0.5 mm or more, the X-ray non-transmission function can be exhibited satisfactorily.

### Industrial applicability

In the marker according to the present invention, the X-ray non-transmission section can have a simple configuration, and is advantageously used for an instrument which performs X-ray imaging to confirm the position of the marker within a human body.

In addition, in the stent according to the present invention, the position of the stent can be reliably confirmed when the stent is mounted in a target affected part, which is advantageous.

Further, in the marker and the stent according to the present invention, since the pipe cannot be fallen off the stent, as described above, the stent can have high safety.

## Claims

1. A stent (C) with a marker (A, B) for confirming a position of the stent (C) within a human body, the stent formed of wires, wherein the stent (C) has a stent main wire (1) formed in a zigzag pattern, and wherein a pipe (10, 11, 12), which is made of a raw material which can be recognized by at least one of an X-ray imaging apparatus and a magnetic resonance imaging (MRI) apparatus is fitted to a zigzag-shaped turn-back section (E) of the stent main wire (1) and/or a vicinity of the turn-back section (E), **characterized in that** the pipe (10, 11, 12) as the marker (A, B) has a width of at least 2,5 mm, a length of at least 1,0 mm and a thickness of at least 0,5 mm.

2. The stent (C) according to claim 1, wherein the turn-back section (E) has a curved section (1a) formed in a curved shape, and a parallel section (1b) which is continuous with the curved section (1a), and the pipe (10, 11, 12) is fitted to the parallel section (1b).

3. The stent (C) according to claim 1 or 2, wherein the pipe (10, 11, 12) is fitted to each of the stent main wires (1) configuring the zigzag-shaped turn-back section (E) and/or the turn-back section (E) vicinity, and/or is fitted so as to hold each of the stent main wires (1) together.

4. The stent (C) according to claim 3, wherein the pipe (10, 11, 12) is fitted so as to hold each of the stent main wires (1) configuring the zigzag-shaped turn-back section (E) or the turn-back section vicinity together by including a different wire arranged so as to extend from the turn-back section and/or the turn-back section vicinity (E).

5. The stent (C) according to any one of claims 1 to 4, wherein the pipe (10, 11, 12) is fitted so as to be fixed to at least one wire configuring the stent (C).

## Patentansprüche

1. Stent (C) mit einem Marker (A,B) zur Bestätigung einer Position des Stents (C) in einem menschlichen Körper, wobei der Stent aus Drähten geformt ist, wobei der Stent (C) einen in einem Zick-Zack-Muster geformten Stent-Hauptdraht (1) hat und wobei ein Rohr (10,11,12), das aus einem Rohmaterial geformt ist, das mindestens durch eine der Vorrichtungen Röntgenbildgebungsapparat oder Magnetresonanztomographieapparat erkennbar ist, an einen zick-zack-förmigen Umkehrabschnitt (E) des Stent-Hauptdrahts (1) und/oder eine Umgebung des Umkehrabschnitts (E) angepasst ist,
**dadurch gekennzeichnet, dass**
das Rohr (10,11,12) als Marker (A,B) eine Weite von mindestens 2,5 mm, eine Länge von wenigstens 1,0 mm und eine Dicke von mindestens 0,5 mm aufweist.

2. Stent (C) nach Anspruch 1, wobei der Umkehrabschnitt (E) einen gebogenen Abschnitt (1a) , der in gebogener Form geformt ist und einen parallelen Abschnitt (1b), der mit dem gebogenen Abschnitt zusammenhängt, aufweist, und dass das Rohr (10,11,12) an den parallelen Abschnitt (1b) angepasst ist.

3. Stent (C) nach Anspruch 1 oder 2, wobei das Rohr (10,11,12) an jeden der Stent-Hauptdrähte (1), die den zick-zack-förmigen Umkehrabschnitt (E) und/oder die Umgebung des zick-zack- förmigen Umkehrabschnitts (E) konfigurieren, angepasst ist, und/oder derart angepasst ist, dass es jeden der Stent-Hauptdrähte (1) zusammenhält.

4. Stent (C) nach Anspruch 3, wobei das Rohr (10,11,12) derart angepasst ist, dass es jeden der Stent-Hauptdrähte (1), die den zick-zack-förmigen Umkehrabschnitt (E) und/oder die Umgebung des zick-zack- förmigen Umkehrabschnitts (E) konfigurieren, dadurch zusammenhält, dass es anderen Draht einschließt, der so angeordnet ist, dass er sich von dem Umkehrabschnitt und/oder der Umgebung des Unterabschnitts (E) weg erstreckt.

5. Stent (C) nach einem der Ansprüche 1 bis 4, wobei das Rohr (10,11,12) so angepasst ist, das es an wenigstens einem der Drähte, die den Stent (C) bilden, fixiert ist.

## Revendications

1. Stent (C) comprenant un repère (A, B) permettant de confirmer sa position dans un corps humain, ce stent (C) étant formé de fils métalliques, et comprenant un fil métallique principal de stent (1) mis en forme selon un motif en zigzag et un tube (10, 11, 12) réalisé en un matériau brut pouvant être reconnu par un appareil d'imagerie par rayons X et/ou un appareil d'imagerie par résonance magnétique nucléaire (IRM) étant ajusté sur un segment faisant demi-tour mise en forme en zigzag (E) du fil métallique principal du stent (1), et/ou au voisinage de ce segment (E) faisant demi-tour,
**caractérisé en ce que**
le tube (10, 11, 12) constituant le repère (A, B) a une largeur d'au moins 2,5 mm, une longueur d'au moins 1,0 mm et une épaisseur d'au moins 0,5 mm.

2. Stent (C) conforme à la revendication 1,
dans lequel le segment faisant demi-tour (E) a un segment courbe (1a) réalisé selon une forme courbe et un segment parallèle (1b) qui est situé dans la continuité du segment courbe (1a), et le tube (10, 11, 12) est ajusté sur le segment parallèle (1b).

3. Stent (C) conforme à la revendication 1 ou 2,
dans lequel le tube (10, 11, 12) est ajusté sur chacun des fils métalliques principaux (1) du stent constituant le segment faisant demi-tour en forme de zigzag (E) et/ou au voisinage du segment faisant demi-tour (E), et/ou est ajusté pour maintenir ensemble chacun des fils métalliques principaux (1) du stent.

4. Stent (C) conforme à la revendication 3,
dans lequel le tube (10, 11, 12) est ajusté pour maintenir ensemble chacun des fils métalliques principaux du stent (1) constituant le segment faisant demi-tour en forme de zigzag (E) ou le voisinage du segment faisant demi-tour, en renfermant un fil métallique différent agencé pour s'étendre à partir du segment faisant demi-tour et/ou du voisinage de ce segment faisant demi-tour (E).

5. Stent (C) conforme à l'une quelconque des revendications 1 à 4,
dans lequel le tube (10, 11, 12) est ajusté pour être fixé à au moins un fil métallique constituant le stent (C).
